(19) Europäisches Patentamt
European Patent Office
Office européen des brevets

(11) **EP 3 669 845 A1**

(12) **EUROPEAN PATENT APPLICATION**

(43) Date of publication:
**24.06.2020 Bulletin 2020/26**

(51) Int Cl.:
***A61F 13/15*** *(2006.01)*     ***A61F 13/514*** *(2006.01)*
***A61F 13/84*** *(2006.01)*     ***G01N 21/35*** *(2014.01)*

(21) Application number: **19217286.4**

(22) Date of filing: **18.12.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **19.12.2018 US 201862781676 P**

(71) Applicant: **The Procter & Gamble Company
Cincinnati, OH 45202 (US)**

(72) Inventor: **Vetter, Nicholas David
Cincinnati, Ohio 45202 (US)**

(74) Representative: **P&G Patent Germany
Procter & Gamble Service GmbH
Sulzbacher Straße 40
65824 Schwalbach am Taunus (DE)**

(54) **ABSORBENT ARTICLE COMPRISING PRINTED REGION**

(57) An absorbent article includes a topsheet, backsheet and an absorbent core disposed between the topsheet and backsheet; and an infrared-transparent printed region. The infrared-transparent printed region includes a colorant. The colorant includes carbon black and comprises a L* value of about 85 or less. The printed region is fluorescent-free.

FIG. 1

**Description**

FIELD OF THE INVENTION

The present disclosure relates to absorbent articles with printed regions, more particularly, absorbent articles having printed regions containing carbon black pigments and related methods for manufacturing and inspecting said articles.

BACKGROUND OF THE INVENTION

Many current products, such as diapers and training pants, include printed designs to improve their appearance. It is well known that consumers both appreciate and seek out absorbent articles that have colors or graphics. Indeed, certain colors convey information, such as functionality or comfort to consumers. For instance, in absorbent articles, various colors (e.g., grays) may be used to convey softness or identify areas of the article where functional components exist (e.g., provide a graphic outline superimposing the absorbent core). Further, certain colors (e.g., shades of black) may provide definition and apparent dimensionality to design elements.
Manufacturers often weigh providing suitable color to their products with process requirements. For instance, manufacturers may use inspection equipment to ensure that articles are assembled correctly, to identify contaminants and/or to otherwise identify defective items on the line. However, these inspection systems are sometimes falsely triggered by pigments used for graphics. Indeed, inspection systems often transmit radiation waves, such as infrared radiation, through an inspection zone of a product. The systems further include component(s) which sense the transmitted light waves and form an image. The resulting image is darkened in areas where the transmitted radiation waves have been absorbed. Typically, the materials forming a product (e.g., substrates in an absorbent article) will absorb the transmitted light, and ideally inks and printing will not absorb such transmitted light. In this way, the layers and substrates etc. of a product can be seen in the image without regard to graphics disposed thereon. However, some pigments also absorb the system's transmitted radiation, which impedes a manufacturer's ability to detect the placement and position of substrates. In other words, the pigment results in darkened areas on the inspection image, indicating that the product may be defective. Likewise, other components in inks may interfere with inspection systems. For example, formulations that include components that emit radiation could interfere with the desired detection.
Therefore, there is a need for methods and articles that include ink formulations that do not interfere with inspection systems. Further, there is a continued need to provide printing with desired colors to convey functionality, performance and/or comfort. There is also a continued need to utilize inexpensive and easily accessible materials for inks and printing as well as provide cost effective and efficient means for balancing printing needs with inspection systems.

SUMMARY OF THE INVENTION

The present invention relates to articles having printed regions that comprise carbon black and are infrared-transparent. An absorbent article may comprise a topsheet, a backsheet and an absorbent core disposed between the topsheet and the backsheet. The absorbent article may further comprise an infrared-transparent printed region. The infrared-transparent printed region may comprise a colorant. The colorant includes a carbon black pigment and comprises a $L^*$ value of about 85 or less. The infrared-transparent printed region may also be fluorescent-free. Additionally, or alternatively, the colorant may comprise one or more non-IR absorbing components, such as non-IR absorbing pigments. In nonlimiting examples, the colorant may be within the boundary described by the following system of equations:

$$\{a^* = -1 \, to \, 3; b^* = 12 \, to \, 6\} \rightarrow b^* = -1.5a^* + 10.5$$

$$\{a^* = 3 \, to \, 7; b^* = 6 \, to \, 4\} \rightarrow b^* = -0.5a^* + 7.50$$

$$\{a^* = 7 \, to \, 8; b^* = 4 \, to \, 0\} \rightarrow b^* = -4\,a^* + 32$$

$$\{a^* = 8 \, to \, 0; b^* = 0 \, to -10\} \rightarrow b^* = 1.25a^* - 10$$

$$\{a^* = 0\ to\ -7; b^* = -10\ to\ -8\} \rightarrow b^* = -0.29a^* - 10$$

$$\{a^* = -7\ to\ -9; b^* = -8\ to\ 1\} \rightarrow b^* = -4.5a^* - 39.5$$

$$\{a^* = -9\ to\ -1; b^* = 1\ to\ 12\} \rightarrow b^* = 1.375a^* + 13.38$$

wherein L* is 20 to 50.

In other embodiments, a method of manufacturing an absorbent article having a printed region includes printing an article component in an inspection zone with a colorant. The colorant may be fluorescent-free. The colorant comprises a carbon black pigment and a L* value of 85 or less. The method further includes passing the inspection zone through an infrared inspection system.

BRIEF DESCRIPTION OF THE DRAWINGS

Fig. 1 is a plan view of an exemplary embodiment of an absorbent article as detailed herein. The absorbent article is shown in a flat, uncontracted state. The wearer-facing side is facing the view.

Fig. 2 is a plan view of an exemplary embodiment of an absorbent article as detailed herein. The absorbent article is shown in a flat, uncontracted state. The garment-facing side is facing the view.

Fig. 3A is a perspective view of a first surface of a substrate according to another nonlimiting embodiment of the present invention.

Fig. 3B is a plan view of a second surface of a substrate according to a nonlimiting embodiment of the present invention.

Fig. 4A is a color space graph.

Fig. 4B is another color space graph.

Fig. 5 is a schematic representation of a method for inspecting according to a nonlimiting embodiment of the present invention.

Fig. 6 is a cross-sectional view of the absorbent article taken about the lateral centerline in Fig. 1 in accordance with a non-limiting embodiment of the present invention.

Fig. 7a is a perspective view of an exemplary absorbent pant according to one nonlimiting embodiment of the present invention.

Fig. 7b is a plan view of an exemplary absorbent pant precursor structure, prior to joining of the front and rear sections of the belt.

Fig. 8 is a perspective view of a package in accordance with one embodiment of the present invention.

DETAILED DESCRIPTION OF THE INVENTION

"Disposable," in reference to absorbent articles, means that the absorbent articles are generally not intended to be laundered or otherwise restored or reused as absorbent articles (i.e., they are intended to be discarded after a single use and, preferably, to be recycled, composted or otherwise discarded in an environmentally compatible manner).

"Absorbent article" refers to devices which absorb and contain body exudates and, more specifically, refers to devices which are placed against or in proximity to the body of the wearer to absorb and contain the various exudates discharged from the body. Exemplary absorbent articles include diapers, training pants, pull-on pant-type diapers (i.e., a diaper having a pre-formed waist opening and leg openings such as illustrated in U.S. Patent No. 6,120,487), refastenable diapers or pant-type diapers, incontinence briefs and undergarments, diaper holders and liners, feminine hygiene garments such as panty liners, absorbent inserts, and the like.

"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).

"Colorant" refers to a color-producing species, which may comprise one or more of pigments and/or dyes.

"Design element" as used herein means a shape or combination of shapes that visually create a distinct and discrete component, regardless of the size or orientation of the component. A design element may be present in one or more patterns. A design element may be present one or more times within one pattern. In one nonlimiting example, the same design element is present twice in one pattern - the second instance of the design element is smaller than the first instance. One of skill in the art will recognize that alternative arrangements are also possible. Design elements may comprise insignia. Design elements and/or combinations of design elements may comprise letters, words and/or graphics such as flowers, butterflies, hearts, character representations and the like. Design elements and/or combinations of design elements may comprise instructional indicia providing guidance or instruction to the caregiver relative to placement and/or fit of the article about the wearer.

"Insignia" as used herein means objects, character representations, words, colors, shapes or other indicia that can be used to distinguish, identify or represent the manufacturer, retailer, distributor or brand of a product, including but not limited to trademarks, logos, emblems, symbols, designs, figures, fonts, lettering, crests or similar identifying marks.

"Infrared-transparent" refers to an element does not absorb light having wavelengths in the range of 700 to 1200 nm, such that an inspection system as described herein does not detect the presence of said element. Stated differently, said element reflects a sufficient amount of light having wavelengths in the range of 700 to 1200 nm such that the inspection system does not recognize the element (e.g., the element is not shown as a darkened area on images produced by the inspection system). The element may reflect more than about 50%, or at least about 60%, or at least about 75%, or at least about 80%, or from about 50% to about 100% of light having wavelengths in the range of 700 to 1200 nm, which is emitted by the inspection system, reciting for said range every 5% increment therein. In nonlimiting examples, a colorant, pigment, dye, printed region or any combinations of the foregoing may be infrared-transparent.

"Film" means a sheet-like material wherein the length and width of the material far exceed the thickness of the material (e.g., 10x, 50x, or even 1000x or more). Films are typically liquid impermeable but may be configured to be breathable.

"Joined" refers to configurations whereby an element is directly secured to another element by fixedly attaching or removably attaching the element directly to the other element, and configurations whereby an element is indirectly secured to another element by fixedly attaching or removably attaching the element to intermediate member(s) which in turn are fixedly attached or removably attached to the other element.

"Non-IR absorbing," refers to an element that does not absorb light having wavelengths in the range of 700 to 1200 nm. In nonlimiting examples, printed regions, colorants, pigments, dyes or any combinations of the foregoing may be non-IR absorbing.

"Nonwoven" means a porous, fibrous material made from continuous (long) filaments (fibers) and/or discontinuous (short) filaments (fibers) by processes such as, for example, spunbonding, meltblowing, airlaying, carding, coforming, hydroentangling, and the like. Nonwovens are fibrous substrates which do not have a woven or knitted filament pattern. Nonwovens may be liquid permeable or impermeable.

"Pant" refers to disposable absorbent articles having a pre-formed waist and leg openings. A pant may be donned by inserting a wearer's legs into the leg openings and sliding the pant into position about the wearer's lower torso. Pants are also commonly referred to as "closed diapers", "prefastened diapers", "pull-on diapers", "training pants" and "diaper-pants."

"Pattern" as used herein means a decorative or distinctive design, not necessarily repeating or imitative, including but not limited to the following: clustered, geometric, spotted, helical, swirl, arrayed, textured, spiral, cycle, contoured, laced, tessellated, starburst, lobed, blocks, pleated, concave, convex, braided, tapered, and combinations thereof.

"Substrate" includes any material that the colorants of the present invention can be printed on. Thus, substrates of the present invention include, but are not limited to nonwovens, films, fibrous polyolefin webs, cellulosic webs, laminates of one or more of the above or any combination of one or more of the above.

"Web" means a material capable of being wound into a roll. Webs may be films, nonwovens, laminates, apertured films and/or laminates, and the like.

PRINTED REGION

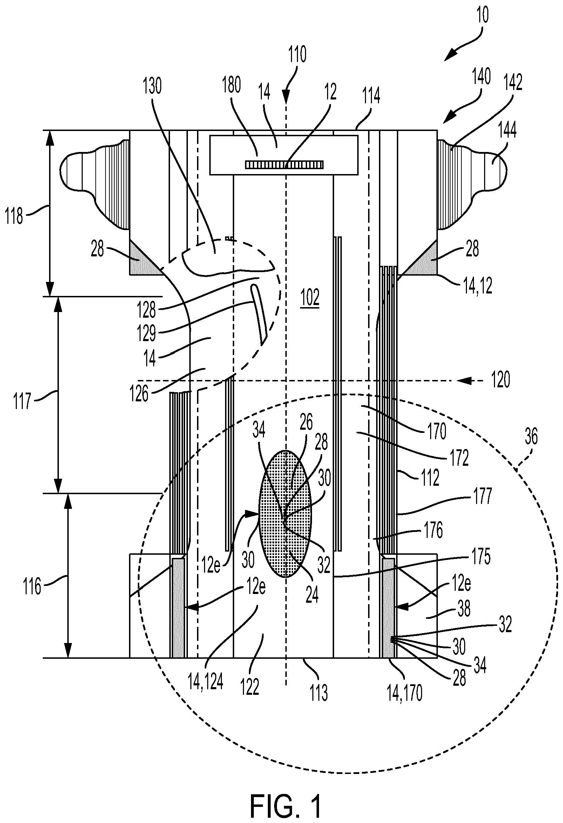

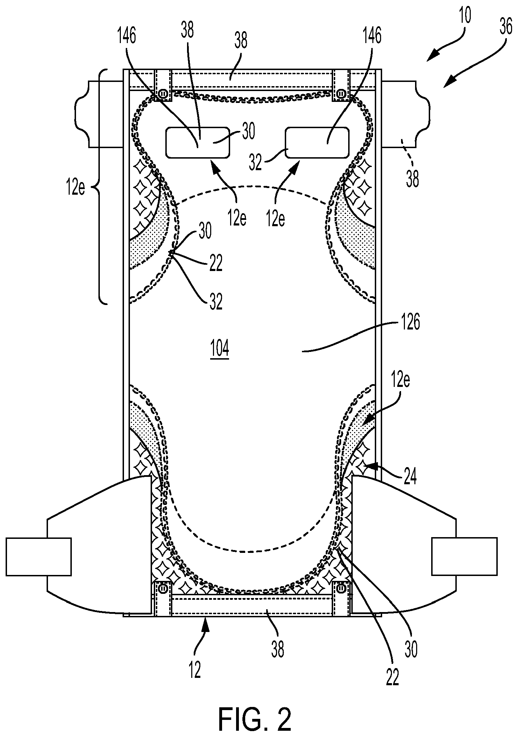

The present invention relates to an article 10 having one or more printed regions 12 disposed on one or more substrates 14, as shown in Figs. 1 and 2. FIG. 1 is a plan view of an exemplary, non-limiting embodiment of an absorbent article 10 of the present invention in a flat, uncontracted state. The body-facing surface 102 of the absorbent article 10 is facing the viewer. FIG. 2 is a plan view of an exemplary, non-limiting embodiment of an absorbent article 10 of the present invention in a flat, uncontracted state. The garment-facing surface 104 of the absorbent article 10 is facing the viewer.

As stated, the article 10 may comprise a substrate 14 having a printed region 12. The substrate 14 may comprise a nonwoven, film, or combinations thereof. Suitable nonwoven substrates include polyolefin-based nonwovens, including but not limited to nonwovens having polypropylene fibers and/or polyethylene fibers and/or bicomponent fibers comprising a polyolefin. Nonlimiting examples of suitable fibers include spunbond fibers, carded fibers, meltblown fibers or combinations thereof. Suitable films include polymeric films, including films formed from polymers comprising styrene derivatives, polyesters, polyurethanes, polyether amides, polyolefins, or combinations thereof. Films may be substantially liquid impermeable, microporous, monolithic and/or apertured. Substrates may be layered, e.g. a SMS nonwoven, a bilayer film, a nonwoven-film laminate. Substrates may be extensible or elastic. Additionally, or alternatively, the substrate may comprise a basis weight of at least about 8 gsm, or about 65 gsm or less, or from about 8 gsm to about 65 gsm, or from about 10 gsm to about 45 gsm, or from about 15 gsm to about 30 gsm, reciting for each range every 1 gsm increment therein.

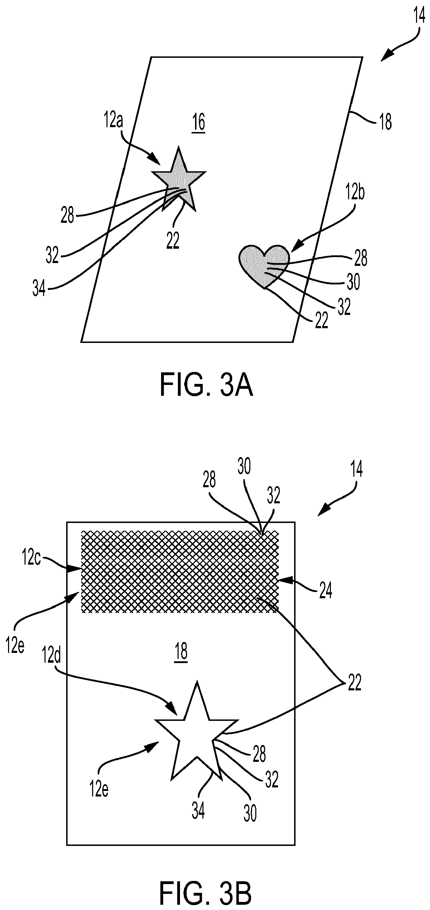

Turning to Fig. 3A, a substrate 14 may comprise a first surface 16 and a second surface 18, the second surface being substantially opposite the first surface 16. The first surface 16 may comprise a printed region 12, or multiple printed regions 12a, 12b. Where the surface 16 comprises more than one printed region, the printed regions 12a, 12b may comprise the same or different features; such features include the aggregate shape, dimensions, design elements, ink type, pigments and/or color. The printed regions 12a, 12b may match in some embodiments. Matching does not require printed regions to be exactly the same; rather, the printed regions may comprise substantially similar design elements, which may be rotated, mirrored, reduced in size, enlarged in size and/or altered in aspect ratio between the printed regions and still be considered matching.

In some embodiments, the second surface 18 comprises one or more additional printed regions 12c as shown in Fig. 3B. Where the second surface 18 comprise more than one printed region, the printed regions 12c, 12d may comprise the same or different features; such features include the aggregate shape, dimensions, design elements, ink type, pigments and/or color. Likewise, a printed region 12 on the first surface 16 may be the same as or may differ from the printed region on a second surface 18. The printed regions on the two surfaces may overlap, partially overlap, or not overlap at all. In some embodiments, the printed regions on the two surfaces match and/or multiple printed regions on the second surface 18 match. The printed regions on the two surfaces may comprise the same colorant or different colorants.

Any printed region 12 may comprise one or more design elements 22, including but not limited to graphics, letters, instructional indicia and insignia. In some embodiments, a printed region 12 comprises a pattern 24, as shown for example in Fig. 3B.

The substrate 14 may be printed using any suitable means, including flexographic printing, inkjet printing, energy curable printing and combinations thereof.

Returning to Fig. 1, it is also contemplated that the printed region 12 may include materials or other than substrates, including but not limited to elastic strand material, absorbent material and adhesives. In some embodiments, the printed region comprises a colored adhesive 26, colored elastic strand 27 and combinations thereof. In nonlimiting examples, colored adhesives may be disposed using patterned slot coating techniques disclosed in commonly assigned U.S. Pat. Pub. Nos. 2014/0148773, 2014/0148774 and 2014/0144579.

The printed region 12 may comprise one or more colorants 28. The colorants may comprise pigments. In some embodiments, the colorant comprises carbon black (reference number 30 in the figures). A suitable carbon black pigment is commercially available from BASF under tradename MICROLITH® Black 0066. The colorant may comprise at least 0.005%, or at least about 0.01%, or at least 0.05%, or at least 0.1%, or up to about 5%, or up to about 3%, or up to about 1%, or up to about 0.5%, from about 0.005% to about 1%, or from about 0.01% to about 0.5% of carbon black pigment by weight of the colorant, reciting for each range every 0.01% increment therein. Without being bound by theory, it is believed that inclusion of carbon black pigment is desirable due to its ample supply in the market, low cost, and ability to enrich, intensify and/or darken colors at very low levels. Stated differently, adding very low levels of carbon black can noticeably alter the appearance of the colorant. This is especially desirable when darker colors, such as black and gray, or darker tones of any color are needed.

The colorant may further include one or more additional components, which may include dye(s) and/or pigment(s). In certain embodiments, the colorant comprises one or more non-IR absorbing components 32. The non-IR absorbing component may comprise a pigment and/or a dye. In nonlimiting examples, the color comprises a non-IR absorbing pigment. Suitable non-IR absorbing components include azo dyes, Solvent Yellow 14, Dispersed Yellow 23, Metanil Yellow, Solvent Green 4, Acid Red 52, Basic Red 1, Solvent Orange 63, azine dyes, dairylide yellow AAOT, Pigment Yellow 14, dairylide yellow AAOA, Pigment Yellow 12, lithol red, Pigment Red 52, toluidine red, Pigment Red 22, dioxazine violet, Pigment Violet 23, naphthoic acid red, Pigment Red 48:2, titanium dioxide, Pigment White 6, iron oxides, yellow iron oxides, brown iron oxides, Pigment Black 11, chromium oxide, green chromium oxide, ferric ammonium ferrocyanide, Blue ferric ammonium ferrocyanide, Pigment Black 32, and PALIOGEN® Black L 0086 (available from BASF Corporation). Suitable non-IR absorbing pigments and/or dispersions are also disclosed in U.S. Pat. App. Nos. 8,674,006 and 9,217,070.

The colorant may be a fluorescent-free colorant 34. Likewise, a printed region may be a fluorescent-free region 12e. By "fluorescent-free," it is meant that the colorant or region, respectively, does not contain fluorescors or other ingredients which emit visible, infrared or fluorescent light in response to exposure to radiation or visible light. Without being bound by theory, it is believed that fluorescents may interfere with inspection systems (discussed below) because the fluorescents may emit a variable amount of infrared light. The emitted light may be mistaken by a sensor as an area of an article which does not absorb light transmitted by the system, and therefore the resulting image may be distorted.

In some embodiments, the colorant comprises cyan, magenta, yellow, black or combinations thereof. In non-limiting examples, the colorant may comprise black. In further nonlimiting examples, the colorant may comprise gray. The colorant may comprise a L* value on the CIE L*a*b* color space model of about 85 or less, or about 75 or less, or about 70 or less, or about 65 or less, or about 50 or less, or about 45 or less, or about 20 or less, or from about 5 to about 85, or from about 10 to about 75, from about 15 to about 70, or from about 20 to about 65, reciting for each range every 0.5 increment therein, as determined by the Color Method herein. Additionally, or alternatively, the colorant may comprise an a* value on the CIE L*a*b* model of from about -9 to about 9, or from about -4 to about 4, or from about -2 to about 2.5, or from about -1 to about 2, reciting for each interval every 0.5 increment therein, as determined by the Color Method herein. Additionally, or alternatively, the colorant may comprise a b* value on the CIE L*a*b* model of from about -10 to about 10, or from about -7 to about 7, or from about -5 to about 5.5, or from about -4 to about 5, reciting for each interval every 0.5 increment therein, as determined by the Color Method herein.

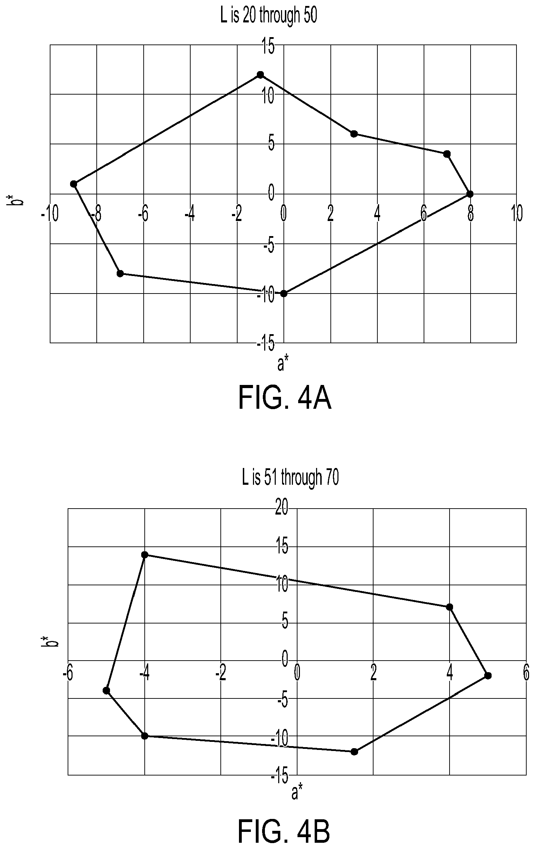

In certain embodiments, the colorant comprises a color having coordinates within the boundary defined by the following system of equations and illustrated in Fig. 4A:

$$\{a^* = -1 \ to \ 3; b^* = 12 \ to \ 6\} \rightarrow b^* = -1.5a^* + \ 10.5$$

$$\{a^* = 3 \ to \ 7; b^* = 6 \ to \ 4\} \rightarrow b^* = -0.5a^* + \ 7.50$$

$$\{a^* = 7 \ to \ 8; b^* = 4 \ to \ 0\} \rightarrow b^* = -4 \ a^* + 32$$

$$\{a^* = 8 \ to \ 0; b^* = 0 \ to - 10\} \rightarrow b^* = 1.25a^* - 10$$

$$\{a^* = 0 \ to - 7; b^* = -10 \ to - 8\} \rightarrow b^* = -0.29a^* - 10$$

$$\{a^* = -7 \ to - 9; b^* = -8 \ to \ 1\} \rightarrow b^* = -4.5a^* - 39.5$$

$$\{a^* = -9 \ to - 1; b^* = 1 \ to \ 12\} \rightarrow b^* = 1.375a^* + 13.38$$

wherein L* is 20 to 50.

In further embodiments, the colorant comprises a color having coordinates within the boundary defined by the following system of equations and illustrated in Fig. 4B:

$$\{a^* = -4 \ to \ 4; b^* = 14 \ to \ 7\} \rightarrow b^* = -0.875a^* + \ 10.5$$

$$\{a^* = 4 \ to \ 5; b^* = 7 \ to - 2\} \rightarrow b^* = -9a^* + 43$$

$$\{a^* = 5 \ to \ 1.5; b^* = -2 \ to - 12\} \rightarrow b^* = 2.9 \ a^* - 16.3$$

$$\{a^* = 1.5 \ to - 4; b^* = -12 \ to - 10\} \rightarrow b^* = -0.36a^* - 11.5$$

$$\{a^* = -4 \ to - 5; b^* = -10 \ to - 4\} \rightarrow b^* = -6a^* - 34$$

$$\{a^* = -5\ to - 4; b^* = -4\ to\ 14\} \rightarrow b^* = 18a^* + 86$$

wherein L* is 51 to 70. The L*, a* and b* values may be determined using the Color Method herein.

Without being bound by theory, it is believed that colors within the aforementioned color spaces provide greater definition to printed areas, making them more apparent to consumers, as well as adding perceived three-dimensionality to the printed areas. Further, colors within the aforementioned color spaces may be used to identify functional components of articles (e.g., areas of enhanced absorbency, areas of stretch, areas where fastening tabs are to be secured). The colors within the aforementioned color spaces may also serve to provide consumers with perception of softness and indicate that the article will perform as intended.

Colorants used in the present invention may be incorporated into, or in the form of, solvent-based inks, energy curable inks, and combinations thereof.

The printed region 12 may be infrared-transparent. The infrared-transparent region may comprise a carbon black pigment, which is known to absorb infrared light waves.

Returning to Figs. 1 and 2, the article 10 may comprise an inspection zone 36. One or more printed regions may be disposed within an inspection zone. The inspection zone may comprise any portion of the article which a manufacturer desires to check for product quality. For example, the inspection zone may include areas inspected for contaminants, proper positioning of article components, angles of substrate edges, absence or existence of article components 38, and component sizes and shapes. The inspection zone may comprise an entire substrate 14, the entire first surface of a substrate 16, the entire second surface of substrate 18, portions of any of foregoing or combinations thereof. The inspection zone may comprise the entire assembled article 10 as shown in Fig. 2. The inspection zone may comprise portions of the article joined to one another, such as laminates, seams or abutting edges. The inspection zone may comprise one or more edges of a substrate.

In certain embodiments, a method includes printing an article component 38 in the inspection zone 36. The inspection zone 36 may include any of the features discussed above with respect to inspection zones. Likewise, printing may include using a colorant 28 having any of the features described herein with respect to colorants 28. The colorant 28, for example, may include carbon black pigment 30 and/or may be fluorescent-free. The article component 38 may comprise a substrate 14, as described herein. The article component may comprise portion or portions of a backsheet 126, an ear 140, a leg cuff 170, fastening system 142, landing zone 176, a waist feature 180, a belt 220, seam 224, and combinations thereof. Each of the aforementioned components are described in more detail below.

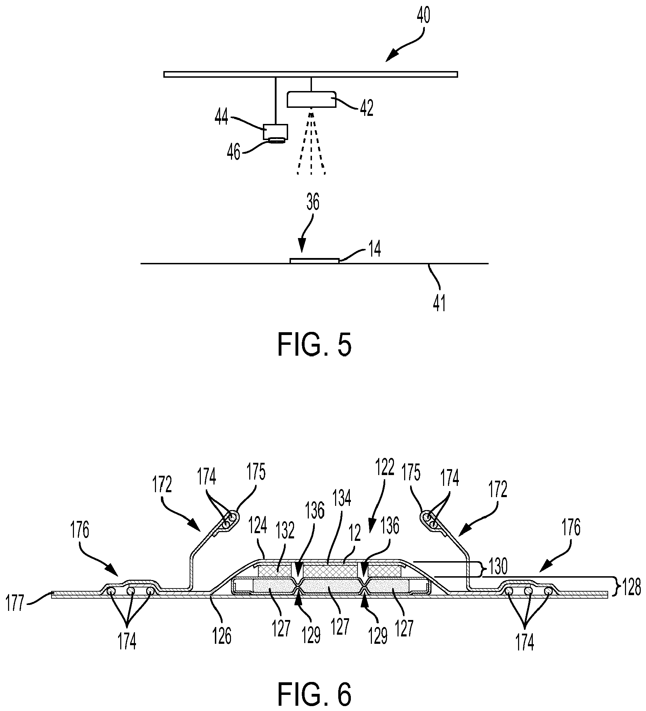

As schematically illustrated in Fig. 5, the method further comprises passing the inspection zone 36 through an infrared inspection system 40. The article component may be moved along a continuous belt 41 as it passes through the system. The inspection system may include a light source 42 which transmits infrared light waves. Suitable infrared inspection systems include systems which transmit infrared light in the range of about 700 nm to about 1200 nm. In nonlimiting examples, the infrared inspection system may comprise a near-infrared system, which transmits infrared light in the range of about 700 nm to about 1000 nm. The infrared inspection system may further include an infrared detection element 44 to detect reflected infrared waves from the inspection zone, as is known in the art. Suitable inspection systems that may utilize infrared radiation are disclosed in U.S. Patent Nos. 8,145,344; 8,145,343; 8,145,338 and 9,910,429.

The method may further include capturing an image of the inspection zone using infrared inspection system. The image may be a thermal image and may be taken by an image capturing device 46, which may be part of the system. The image may be captured for any suitable amount of time. In some embodiments, the image is displayed on a monitor and/or printed, as is known in the art.

ARTICLES COMPRISING THE PRINTED REGION

As stated, the printed region 12 may be disposed on a portion or portions of an absorbent article 10. The absorbent article 10 which may be disposable. Fig. 1 is a plan view of an exemplary, non-limiting embodiment of an absorbent article 10 of the present invention in a flat, uncontracted state. The body-facing surface 102 of the absorbent article is facing the viewer. The absorbent article 10 includes two longitudinal edges 112, a front waist edge 113 opposite a back waist edge 114, and a longitudinal centerline 110 and a lateral centerline 120. The absorbent article 10 comprises a chassis 122. The absorbent article 10 and chassis 122 are shown to have a first waist region 116, a second waist region 118 opposed to the first waist region 116, and a crotch region 117 located between the first waist region 116 and the second waist region 118. The waist regions 116 and 118 generally comprise those portions of the absorbent article 10 which, when worn, encircle the waist of the wearer. The crotch region 117 is the portion of the absorbent article 10 which, when the absorbent article 10 is worn, is generally positioned between the legs of the wearer.

The chassis 122 may comprise a liquid permeable topsheet 124, a backsheet 126, and an absorbent core 128 between the topsheet 124 and the backsheet 126. The topsheet 124 may be joined to the core 128 and/or the backsheet 126. The backsheet 126 may be joined to the core 128 and/or the topsheet 124. It should be recognized that other structures, elements, or substrates may be positioned between the core 128 and the topsheet 124 and/or backsheet 126, including but not limited to an acquisition-distribution system 130. In certain embodiments, the chassis 122 comprises the main structure of the absorbent article 10 with other features added to form the composite absorbent article structure. While the topsheet 124, the backsheet 126, and the absorbent core 128 may be assembled in a variety of well-known configurations, absorbent article configurations are described generally in U.S. Patent Nos. 3,860,003; 5,151,092; 5,221,274; 5,554,145; 5,569,234; 5,580,411; and 6,004,306.

The article 10 may comprise a component 38 having the printed region 12 comprising the colorant 28 as described herein. The article may have a substrate 14, which may be disposed in one of the first waist region, second waist region, and/or crotch region. The substrate 14 may comprise printed regions 12 on one or both surfaces 16, 18. Nonlimiting examples of components comprising a printed region 12 include the topsheet 124, the backsheet 126, the acquisition-distribution system 130, a leg cuff 172, an ear 140, a landing zone 146, a waist feature 180 and a belt 220 and combinations thereof. In some embodiments, the printed region 12 may be disposed on at least 5%, or at least 10%, or at least 20 %, or at least 25%, or from about 10% to about 90% of a surface of the component based on the total area of the surface of said component as determined by the Percent Printed Color Area Test Method herein. The printed region 12 may be disposed on multiple components. In nonlimiting examples, a portion of the printed region is disposed on one component and another portion is disposed on a different component, such that the complete region, complete design element, and/or complete pattern is provided when the separate components are positioned as intended on the final article.

The inspection zone may include multiple components 38, including areas where components are joined together. Inspection zones may include layers of materials, and inspection systems may be used to detect the relative positioning of multiple components. By way of nonlimiting example, the inspection zone may include the landing zone and a portion of the backsheet surrounding the landing zone as shown in Fig. 2. The inspection system may be used to determine if the landing zone is disposed in proper relation to the backsheet or an edge of the backsheet. The skilled person will recognize that inspection zones and systems may be used for various detection purposes.

TOPSHEET:

The topsheet 124 may be positioned at least in partial contact or close proximity to a wearer. Suitable topsheets 124 may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. In certain embodiments, the topsheet 124 comprises a nonwoven. The topsheet 124 is generally supple, soft feeling, and non-irritating to a wearer's skin. Generally, at least a portion of the topsheet 124 is liquid pervious, permitting liquid to readily penetrate through the thickness of the topsheet 124. One topsheet 24 useful herein is available from BBA Fiberweb, Brentwood, TN as supplier code 055SLPV09U. The topsheet 124 may be apertured.

Any portion of the topsheet 124 may be coated with a lotion or skin care composition as is known in the art. Non-limiting examples of suitable lotions include those described in U.S. Patent Nos. 5,607,760; 5,609,587; 5,635,191; and 5,643,588. The specific examples are not limiting, as any lotion or skin care composition known in the art may be utilized. The topsheet 124 may be fully or partially elasticized or may be foreshortened so as to provide a void space between the topsheet 124 and the core 128. Exemplary structures including elasticized or foreshortened topsheets are described in more detail in U.S. Patent Nos. 4,892,536; 4,990,147; 5,037,416; and 5,269,775.

In some embodiments, the topsheet 124 comprises one or more printed regions 12 and/or one or more printed regions are visible when viewing the topsheet though they may be printed on a layer below the topsheet, as is shown in Fig. 1. The printed region 12 (on the topsheet or elsewhere) may comprise colorants 28, which may be disposed in one or more patterns 24 and/or design elements 22 including but not limited to graphics, instructional indicia, and insignia. The inspection zone 36 may include the topsheet or a portion of the topsheet.

ABSORBENT CORE:

Turning to Fig. 6, the absorbent core 128 may comprise a wide variety of liquid-absorbent materials 127 commonly used in disposable diapers and other absorbent articles. Examples of suitable absorbent materials include comminuted wood pulp, which is generally referred to as air felt creped cellulose wadding; melt blown polymers, including co-form; chemically stiffened, modified or cross-linked cellulosic fibers; tissue, including tissue wraps and tissue laminates; absorbent foams; absorbent sponges; superabsorbent polymers; absorbent gelling materials (AGM); or any other known absorbent material or combinations of materials. In one embodiment, at least a portion of the absorbent core is substantially cellulose free and contains less than 10% by weight cellulosic fibers, less than 5% cellulosic fibers, less than 1% cellulosic fibers, no more than an immaterial amount of cellulosic fibers or no cellulosic fibers. It should be understood that an immaterial amount of cellulosic material does not materially affect at least one of the thinness, flexibility, and absorbency of the portion of the absorbent core that is substantially cellulose free. Among other benefits, it is believed that when at least a portion of the absorbent core is substantially cellulose free, this portion of the absorbent core is significantly thinner and more flexible than a similar absorbent core that includes more than 10% by weight of cellulosic fibers. The amount of absorbent material, such as absorbent particulate polymer material present in the absorbent core may vary, but in certain embodiments, is present in the absorbent core in an amount greater than about 80% by weight of the absorbent core, or greater than about 85% by weight of the absorbent core, or greater than about 90% by weight of the absorbent core, or greater than about 95% by weight of the core. The absorbent material 127 may be at least partially surrounded by a core wrap. In some embodiments, the core may comprise one or more channels 129, which are substantially free of absorbent material. In one nonlimiting example, one or more channels may extend longitudinally. Nonlimiting exemplary absorbent structures for use as the absorbent core 128 are described in U.S. Patent No. 4,610,678; 5,260,345; 5,387,207; 5,397,316; 5,625,222, 8,979,815, 9,060,904, and 9,072,634; and U.S. Pat. App. No. 13/491,642.

Articles of the present invention may comprise an Average Number of Particles per Pad of 20 or less, or 15 or less, or 10 or less as determined by the Topsheet AGM Residue (TAGMR) Method herein. In nonlimiting examples, articles of the present invention may comprise a Percentage of Pads with No Residue Particles of at least 20%, or at least 50%, or from 10% to 100% reciting for said range every 10% increment therein as determined by the TAGMR Method herein. In this way, the absorbent material is kept away from the skin of the wearer.

In some embodiments, the absorbent core 128, or a portion of the core such as the core wrap, comprises one or more printed regions 12, having one or more colorants 28. Further, layers above or below the core 128 may comprise a printed region 12. Additionally, or alternatively, the inspection zone 36 may include the absorbent core or a portion of the absorbent core.

BACKSHEET:

The backsheet 126 is generally positioned such that it may be at least a portion of the garment-facing surface 104 of the absorbent article 10. Backsheet 126 may be designed to prevent the exudates absorbed by and contained within the absorbent article 20 from soiling articles that may contact the absorbent article 20, such as bed sheets and undergarments. The backsheet 126 is impervious to liquids. Suitable backsheet 126 materials include films such as those manufactured by Tredegar Industries Inc. of Terre Haute, IN and sold under the trade names X15306, X10962, and X10964. Other suitable backsheet 26 materials may include breathable materials that permit vapors to escape from the absorbent article 10 while still preventing exudates from passing through the backsheet 126. Exemplary breathable materials may include materials such as woven webs, nonwoven webs, polymeric films such as thermoplastic films of polyethylene or polypropylene, composite materials such as film-coated nonwoven webs, and microporous films such as manufactured by Mitsui Toatsu Co., of Japan under the designation ESPOIR NO and by EXXON Chemical Co., of Bay City, TX, under the designation EXXAIRE. Suitable breathable composite materials comprising polymer blends are available from Clopay Corporation, Cincinnati, OH under the name HYTREL blend P18-3097. Such breathable composite materials are described in greater detail in PCT Application No. WO 95/16746 and U.S. Patent No. 5,865,823. Other breathable backsheets including nonwoven webs and apertured formed films are described in U.S. Patent No. 5,571,096. An exemplary, suitable backsheet is disclosed in U.S. Patent No. 6,107,537. Other suitable materials and/or manufacturing techniques may be used to provide a suitable backsheet 126 including, but not limited to, surface treatments, particular film selections and processing, particular filament selections and processing, etc. In one nonlimiting example, the backsheet is a thermoplastic film having a thickness of from about 0.012 mm to about 0.051 mm.

Backsheet 126 may also consist of more than one layer. The backsheet 126 may comprise an outer cover and an inner layer. The outer cover may be made of a non-woven material. The inner layer may be made of a substantially liquid-impermeable film, such as a polymeric film. The outer cover and an inner layer may be joined together by adhesive or any other suitable material or method. A particularly suitable outer cover is available from Corovin GmbH, Peine, Germany as supplier code A18AH0, and a particularly suitable inner layer is available from RKW Gronau GmbH, Gronau, Germany as supplier code PGBR4WPR. While a variety of backsheet configurations are contemplated herein, it would be obvious to those skilled in the art that various other changes and modifications can be made without departing from the spirit and scope of the invention.

In some embodiments shown in Fig. 2, the backsheet 126 comprises one or more printed regions 12 and/or one or more printed regions that are visible when viewing the backsheet though they are printed on a layer above the backsheet. The printed region 12 (on the backsheet or elsewhere) may comprise one or more colorants 28, which may be disposed in one or more patterns 24 and/or design elements 22 including but not limited to graphics, instructional indicia, and insignia. Additionally, or alternatively, the inspection zone 36 may comprise the backsheet or a portion of the backsheet.

ACQUISITION-DISTRIBUTION SYSTEM (ADS):

Referring again to Fig. 6, the absorbent article may comprise an ADS 130. One function of the ADS is to quickly acquire one or more of the fluids and distribute them to the absorbent core in an efficient manner. The ADS may comprise one, two or more layers, which may form a unitary layer or may remain as discrete layers which may be attached to each other. In certain embodiments, the ADS may be substantially free (e.g., 80%, 85%, 90%, 95%, or 99% free of) or completely free of SAP. In nonlimiting examples, the ADS may comprise a distribution layer 132 and/or an acquisition layer 134. In various embodiments, the acquisition layer 134 may acquire bodily exudates and the distribution layer 132 may distribute bodily exudates or both layers may distribute and/or acquire bodily exudates. An ADS disclosed herein may be positioned in an absorbent article: (1) intermediate a liquid pervious material or topsheet and an absorbent core; (2) intermediate an absorbent core and a liquid impervious material or backsheet; or may be otherwise located within the absorbent article. In an embodiment, more than one ADS may be provided in an absorbent article.

In a certain embodiment, the ADS may comprise chemically cross-linked cellulosic fibers. In nonlimiting examples, the distribution layer 132 may comprise at least 50%, or 60%, or 70%, or 80%, or 90%, or even up to 100%, by weight of the layer, of cross-linked cellulose fibers (including the cross-linking agents). The cross-linked cellulosic fibers may be crimped, twisted, or curled, or a combination thereof including crimped, twisted, and curled. Example chemically cross-linked cellulosic fibers are disclosed in US Patent No. 5,137,537. In certain embodiments, the chemically cross-linked cellulosic fibers are cross-linked with between about 0.5 mole % and about 10.0 mole % of a $C_2$ to $C_9$ polycarboxylic cross-linking agent or between about 1.5 mole % and about 6.0 mole % of a $C_2$ to $C_9$ polycarboxylic cross-linking agent based on glucose unit. Citric acid is an example cross-linking agent. In other embodiments, polyacrylic acids may be used. Further, according to certain embodiments, the cross-linked cellulosic fibers have a water retention value of about 25 to about 60, or about 28 to about 50, or about 30 to about 45. A method for determining water retention value is disclosed in U.S. Patent No. 5,137,537. Example chemically cross-linked cellulosic fibers suitable for a distribution layer are disclosed in U.S. Pat. No. 5,549,791, U.S. Pat. No 5,137,537, WO 9534329, or U.S. Pat. App. Publ. No. 2007/118087, U.S. Pat. Publ. No. 2008/0312622 A1.

The distribution layer may typically have an average basis weight of from 30 to 400 g/m$^2$ or from 100 to 300 g/m$^2$, specifically reciting all 1.0 g/m$^2$ increments within the above-specified ranges and any ranges formed therein or thereby. The density of the distribution layer may vary depending on the compression of the absorbent article, but may be between 0.03 to 0.15 g/cm$^3$ or 0.08 to 0.10 g/cm$^3$, specifically reciting all 1.0 g/cm$^3$ increments within the above-specified ranges and any ranges formed therein or thereby, measured at 0.30 psi (2.07kPa).

Additionally, or alternatively, the ADS 130 may comprise an acquisition layer 134. In an embodiment, the acquisition layer 134 may be disposed, for example, between the distribution layer 132 and the topsheet 124. The acquisition layer 134 may comprise a nonwoven, such as an SMS or SMMS material, comprising a spunbond, a melt-blown and a further spunbond layer or alternatively a carded chemical-bonded nonwoven. In some embodiments, the acquisition layer 134 may comprise air or wet-laid cellulosic, cross-linked cellulosic, or synthetic fibers, or blends thereof. In certain embodiments, the acquisition layer 134 may comprise a roll-stock web of synthetic fibers (which may be processed to increase void space, such as by solid state formation), or a combination of synthetic and cellulosic fibers, bonded together to form a high loft material. Alternatively, the acquisition layer 134 may comprise absorbent open cell foam. The nonwoven material may be latex bonded. Example acquisition layers are disclosed in U.S. Pat. No. 7,786,341. Carded, resin-bonded nonwovens may be used, in particular where the fibers used are solid round or round hollow PET staple fibers (50/50 or 40/60 mix of 6 denier and 9 denier fibers). The acquisition layer 134 may be stabilized by a latex binder, for example a styrene-butadiene latex binder (SB latex).

A further acquisition layer may be used in addition to a first acquisition layer described above. For example, a tissue, nonwoven, or other layer may be placed between the first acquisition layer and the distribution layer. The tissue may have enhanced capillarity distribution properties compared to the acquisition layer described above. The tissue, nonwoven, or other layer and the first acquisition layer may be of the same size or may be of a different size. For example, the tissue, nonwoven, or other layer may extend further in the rear of the absorbent article than the first acquisition layer. An example of hydrophilic tissue is a 13 - 15 gsm high wet strength made of cellulose fibers from supplier Havix.

In some embodiments, one or more layers of the ADS may comprise channels 136. One or more of the channels 136 may be configured to work in concert with one or more channels 129 in the absorbent core 128, as discussed above. Furthermore, channels 136 may also provide increased void space to hold and distribute urine, feces or other body exudates within the absorbent article, leading to reduced leakage and skin contact. Suitable ADS are described in WO 2000/59430, WO 95/10996, U.S. Pat. No. 5,700,254, WO 02/067809, and US Pat. Pub. No. 2015/065973 for example.

In some embodiments, the ADS 130 comprises one or more printed regions 12. The printed region 12 may comprise one or more colorants 28, which may be disposed in one or more patterns 24 and/or design elements 22 including but not limited to graphics, instructional indicia, and insignia. In some nonlimiting examples, the printed region 12 is disposed on the acquisition layer 134. Additionally, or alternatively, the inspection zone 36 may comprise the ADS or a portion of the ADS. The inspection zone 36 may comprise the distribution layer and/or the acquisition layer, portions of the distribution layer and/or acquisition layer, and combinations thereof.

EARS/FASTENERS:

The absorbent article 10 may include front ears and/or back ears 140 as shown in Fig. 1, for example. The ears may be an integral part of the chassis, such as formed from the topsheet 124 and/or backsheet 126 as side panels. Alternatively, the ears may be separate elements attached by gluing, heat embossing, and/or pressure bonding. Each ear may be extensible or inextensible. The ears 140 may be formed from nonwoven webs, woven webs, knitted fabrics, polymeric and elastomeric films, apertured films, sponges, foams, scrims, and combinations and laminates thereof. In some embodiments, the ear may include elastomers (e.g., elastic strands, LYCRA® fibers), such that the ear is stretchable.

The absorbent article 10 may also include a fastening system 142. When fastened, the fastening system 142 interconnects the first waist region 116 and the rear waist region 118 resulting in a waist circumference that may encircle the wearer during wear of the absorbent article 10. The fastening system 142 may comprise a fastener 144 such as tape tabs, hook and loop fastening components, interlocking fasteners such as tabs & slots, buckles, buttons, snaps, and/or hermaphroditic fastening components, although any other known fastening means are generally acceptable. Some exemplary surface fastening systems are disclosed in U.S. Patent Nos. 3,848,594; 4,662,875; 4,846,815; 4,894,060; 4,946,527; 5,151,092; and 5,221,274. An exemplary interlocking fastening system is disclosed in U.S. Patent No. 6,432,098. The fastening system 142 may also provide a means for holding the article in a disposal configuration as disclosed in U.S. Pat. No. 4,963,140. The fastening system 142 may also include primary and secondary fastening systems, as disclosed in U.S. Pat. No. 4,699,622. The fastening system 142 may be constructed to reduce shifting of overlapped portions or to improve fit as disclosed in U.S. Patent Nos. 5,242,436; 5,499,978; 5,507,736; and 5,591,152. In some embodiments, the fastening system 142 and/or the fastener 144 is foldable.

Stretchable ears and/or fastening members may facilitate the attachment of the fastening members to a landing zone 146 and/or maintain the taped diapers in place around the wearer's waist. The landing zone 146 may be a portion of the backsheet 126, or may be a separate substrate 14, such as a nonwoven substrate, joined to the backsheet. In one nonlimiting example, the landing zone 146 is partially disposed on the longitudinal centerline 100. In another non-limiting example, the landing zone 146 is disposed about 2 mm or less from the longitudinal centerline 100, or about 1 mm or less from the longitudinal centerline 100. The landing zone may comprise fastening components (e.g., mechanical closure elements comprising hook and loop fastening materials, adhesive, or other known means).

Extensible ears and/or fastening members may provide a more comfortable and contouring fit by initially conformably fitting the absorbent article to the wearer and sustaining this fit throughout the time of wear well past when absorbent article has been loaded with fluids or other bodily exudates since the elasticized ears allow the sides of the absorbent article to expand and contract. Exemplary ears and/or fastening systems are disclosed in U.S. Patent Nos. 6,863,666; 6132411; 7,870,652; 8,992,499; 8,690,852; 8,382,736.

In some embodiments, an ear 140, a fastening system 142 and/or a landing zone 146 may each independently comprise one or more printed regions 12. The printed regions may comprise one or more colorants 28, which may be disposed in one or more patterns 24 and/or design elements 22 including but not limited to graphics, instructional indicia, and insignia. Additionally, or alternatively, the inspection zone may include a fastening system, a landing zone, an ear, portions of any of the foregoing, and combinations thereof.

LEG GASKETING SYSTEM:

As illustrated in Fig. 1 and Fig. 6, for example, the absorbent article 10 may comprise a leg gasketing system 170 attached to the chassis 122, which may comprise one or more cuffs. The leg gasketing system may comprise a pair of barrier leg cuffs 172. Each barrier leg cuff may be formed by a piece of material which is bonded to the absorbent article so it may extend upwards from a wearer-facing surface of the absorbent article and provide improved containment of fluids and other body exudates approximately at the junction of the torso and legs of the wearer. The barrier leg cuffs are delimited by a proximal edge joined directly or indirectly to the topsheet 124 and/or the backsheet 126 and a free terminal edge 175, which is intended to contact and form a seal with the wearer's skin. In some embodiments, the free terminal edge 175 comprises a folded edge. The barrier leg cuffs 172 extend at least partially between the front waist edge 113 and the rear waist edge 114 of the absorbent article on opposite sides of the longitudinal axis 110 and are at least present in the crotch region. The barrier leg cuffs may be joined at the proximal edge with the chassis of the article by a bond which may be made by gluing, fusion bonding, or a combination of other suitable bonding processes.

The barrier leg cuffs may be integral with the topsheet 124 or the backsheet 126 or may be a separate material joined to the article's chassis. Each barrier leg cuff 172 may comprise one, two or more elastic elements 174 close to the free terminal edge 175 to provide a better seal. Additionally, or alternatively, one or both of the barrier cuffs 172 may comprise a nonwoven. In some embodiments, a barrier leg cuff 172 comprises a printed region 12. The printed region 12 may comprise one or more colorants, which may be disposed in one or more patterns 24 and/or design elements 22 including but not limited to graphics, instructional indicia, and insignia.

In addition to the barrier leg cuffs 172, the article may comprise gasketing cuffs 176, which are joined to the chassis of the absorbent article, in particular to the topsheet 124 and/or the backsheet 126 and are placed externally relative to the barrier leg cuffs 172. The gasketing cuffs 176 may provide a better seal around the thighs of the wearer. A gasketing cuff may comprise a proximal edge and a free terminal edge 177. The free terminal edge 177 may comprise a folded edge. Each gasketing cuff may comprise one or more elastic elements 174 in the chassis of the absorbent article between the topsheet 124 and backsheet 126 in the area of the leg openings. Additionally, or alternatively, one or both of the gasketing cuffs 176 may comprise a nonwoven. In some embodiments, a gasketing leg cuff 176 comprises a printed region 12. The printed region 12 may comprise one or more colorants, which may be disposed in one or more patterns 24 and/or design elements 22 including but not limited to graphics, instructional indicia, and insignia.

All, or a portion of, the barrier leg cuffs and/or gasketing cuffs may be treated with a lotion or another skin care composition. In further embodiments, the leg gasketing system comprises barrier leg cuffs that are integral with gasketing cuffs. Suitable leg gasketing systems which may be part of the absorbent article are disclosed in U.S. Pat. App. No. 62/134,622, 14/077,708; U.S. Pat. Nos. 8,939,957; 3,860,003; 7,435,243; 8,062,279.

WAIST FEATURE

The absorbent article 10 may comprise at least one elastic waist feature 180 that helps to provide improved fit and containment, as shown in Fig. 1. The elastic waist feature 180 is generally intended to expand and contract to dynamically fit the wearer's waist. Elasticized waist features 180 include waistbands, waist cuffs having pockets formed from a portion of the waist feature 180 that is unattached from the chassis 122, and waist panels designed to fit securely about the abdomen of the wearer. Nonlimiting examples of elasticized waist features are disclosed in U.S. Patent App. Nos. 13/490,543; 14/533,472; and 62/134,622. Waist features 180 may be joined to the chassis 122 in the first waist region 116 and/or in the second waist region 118. In some embodiments, a waist feature 180 comprises a nonwoven substrate 14. The waist feature 180 may also comprise a film substrate 14. In further embodiments, the waist feature 180 comprises a printed region 12. The printed region 12 may comprise one or more colorants, which may be disposed in one or more patterns 24 and/or design elements 22 including but not limited to graphics, instructional indicia, and insignia. Additionally, or alternatively, the inspection zone 36 may comprise a waist feature 180 or a portion of the waist feature. In some embodiment, the waist feature 180 comprises a belt 220 which is discussed in more detail below.

ADULT OR BABY PANT ABSORBENT ARTICLES

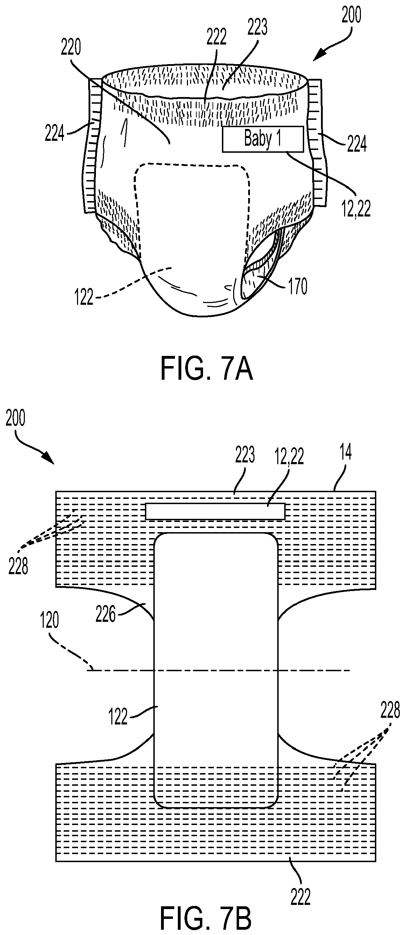

In some embodiments, the article 10 may comprise an absorbent pant 200 as shown in Figs. 7a-7b. The absorbent pant may comprise include a chassis 122, a belt 220 to be positioned about the wearer's waist, and optionally a leg gasketing system 170. Fig. 7b depicts an exemplary precursor structure of the pant in Fig. 7a, in an open configuration laid out flat and stretched out laterally against elastic-induced contraction. In the final assembly of the pant, the front belt portion 222 is joined to rear belt portion 223 at seams 224, which may be permanent or refastenable. To form the pant 200, the precursor structure may be folded at or about lateral centerline 120 with the topsheet 124 facing inward, and the longitudinal edges of the front 222 and rear 223 belt portions may be joined at seams 224, forming a pant structure having leg openings, front waist edge and rear waist edge. In this way, the pant 200 may comprise a pre-formed, continuous waist opening and pre-formed, continuous leg openings for the wearer at the time of donning the pant 200.

The front and rear belt portions 222, 223 may be the outermost structures forming the front and rear regions of a pant 200. The pant may include an outer wrap 226 wrapping the entirety of the front, crotch and rear regions, and forming an outermost pant-shaped structure. In some embodiments, the outer cover of the backsheet forms the outer wrap. Additional layer(s) and elastic members 228 to form front and rear belt portions 222, 223 may be disposed to the inside of outer wrap 226, and be suitably affixed thereto by adhesive lamination, bonding or any other suitable mechanism. An outer wrap 226 may be formed of one or more sections of nonwoven web and may be cut to a profile providing suitably tailored leg opening edge profiles as desired.

Any waist feature 180, including one or both of front and rear belt portions 222, 223 may be formed of layers of nonwoven substrates, which respectively form inner and outer layers. Suitable nonwoven substrates that may be useful in the present invention also include, but are not limited to spunbond, spunlaid, meltblown, spunmelt, solvent-spun, electrospun, carded, film fibrillated, melt-film fibrillated, air-laid, dry-laid, wet-laid staple fibers, and other nonwoven web materials formed in part or in whole of polymer fibers, as known in the art. The nonwoven web may be formed predominately of polymeric fibers. In some examples, suitable non-woven fiber materials may include, but are not limited to polymeric materials such as polyolefins, polyesters, polyamide, or specifically, polypropylene (PP), polyethylene (PE), poly-lactic acid (PLA), polyethylene terephthalate (PET) and/or blends thereof. In some examples, the fibers may be formed of PP/PE blends such as described in U.S. Pat. No. 5,266,392. Nonwoven fibers may be formed of, or may include as additives or modifiers, components such as aliphatic polyesters, thermoplastic polysaccharides, or other biopolymers. Further useful nonwovens, fiber compositions, formations of fibers and nonwovens and related methods are described in U.S. Pat. Nos. 6,645,569; 6,863,933; and 7,112,621; and in U.S. Pat. App. Ser. Nos. 10/338,603; 10/338,610; and 13/005,237. The individual fibers of a nonwoven layer may be monocomponent or multicomponent (including bicomponent). The multicomponent fibers may be bicomponent, with differing polymeric components in, *e.g.,* a core-and-sheath or side-by-side arrangement. The individual components may include polyolefins such as polypropylene or polyethylene, or their copolymers, or polyesters, thermoplastic polysaccharides or other biopolymers.

According to some nonlimiting examples, the nonwoven used for a belt portion may include a material that provides good recovery when external pressure is applied and removed. Further the nonwoven may include a blend of different fibers selected, for example from the types of polymeric fibers described above. In some examples, at least a portion of the fibers may exhibit a spiral curl which has a helical shape. According to one example, the fibers may include bicomponent fibers, which are individual fibers each including different materials, usually a first and a second polymeric material. It is believed that the use of side-by-side bi-component fibers is beneficial for imparting a spiral curl to the fibers. Examples of potentially suitable curled or "crimped" bicomponent fibers and nonwovens formed from them are described in U.S. Pats. Nos. 5,382,400; 5,418,045; 5,707,468; 6,454,989; 6,632,386; 5,622,772 and 7,291,239. For purposes herein, use of a nonwoven formed of crimped bicomponent or multicomponent fibers such as, for example, described in the patents and/or patent applications cited immediately above, may be desired as one or both layers used to form the belt portions, because they can feel particularly soft to the touch (for wearer comfort on the inside and aesthetically pleasing feel on the outside) and are generally quite pliable.

Waist features, such as belt portions, may further comprise one or more elastic members 228. The elastic members 228 may be elastomeric fibers, such as LYCRA® fibers available from INVISTA of Wichita, KS, in various decitex levels. The elastic members 228 may also comprise any heat shrinkable elastic material as is well known in the art. Other suitable elastics can be made various other materials including but not limited to: rubbers, styrene ethylbutylene styrene, styrene ethylene propylene styrene, styrene ethylene propylene styrene, styrene butadiene styrene, styrene isoprene styrene, polyolefin elastomers, elastomeric polyurethanes, and other elastomeric materials known in the art, and combinations thereof. In some nonlimiting examples, the elastic members may be extruded strand elastics with any number of strands (or filaments). In some embodiments, the elastic members can have a decitex ranging from 50 to 2000, or any integer value for any decitex value in this range. However, the skilled person may select the appropriate decitex based on the desired contraction and other principles discussed herein. In further embodiments, the elastic members may be in a form of film. Examples of films have been described in prior patent applications (*see,* for example, U.S. Pat. App. Pub. No. 2010/0040826). The film may be created with a variety of resins combined in at least one of several sublayers, the latter providing different benefits to the film.

In addition, elastic members 228 may take a multitude of configurations. For example, the width may be varied; a single strand or several parallel or non-parallel strands of elastic material may be used; or a variety of shapes may be used including rectilinear and curvilinear; or a variety of cross sectional shapes can be used (circular, rectangular, square, etc.).

Layers of a waist feature (e.g., belt portion) and/or chassis 122 may be joined together about elastic strands 228 by adhesive deposited between the layers, by thermal bonds, by compression bonds, or by a combination thereof. In other examples, the one or more elastic members may be strips or a section of film formed of elastomeric material. Where the elastic member is elongate, it may be desirable that the longer dimension be laterally oriented, or even substantially aligned with the lateral direction, as strands 228 are depicted in Fig. 7b for example.

A belt portion or other form of waist feature may comprise at least 3 waist elastic members, at least 5 elastic members, at least 10 waist elastic members, or at least 15 waist elastic members 228, or from about 2 to about 35 waist elastic members, or from about 5 to about 25 waist elastic members, reciting for each range every 1 increment therein.

In one embodiment, adjacent elastic members 228 are spaced a longitudinal distance of at least 3.5 mm apart from one edge of the member to the other edge of the member, optionally at least 4 mm apart; optionally at least 4.5 mm apart; optionally at least 5 mm apart; optionally at least 5.5 mm apart; optionally at least 6 mm apart; optionally at least 6.5 mm apart; optionally at least 7 mm apart; optionally at least 7.5 mm apart; optionally at least 8 mm apart; optionally at least 8.5 mm apart; optionally at least 9 mm apart; optionally at least 9.5 mm apart; optionally at least 10 mm apart; optionally at least 10.5 mm apart; optionally at least 11 mm apart; optionally at least 11.5 mm apart; optionally at least 12 mm apart. The spacing between elastic members may be the same or different across the longitudinal length of the waist feature. For example, the spacing between adjacent elastic members could uniformly be 7 mm or there could be variable spacing (i.e., two adjacent elastic members are separated by 3 mm, another two are separated by 6.5 mm, etc.).

During manufacture of the waist feature, the elastic members 228 may be pre-strained by a desired amount as they are being incorporated into the waist feature. Upon subsequent relaxation of the waist feature, the elastic members will contract laterally toward their unstrained lengths. This may cause layers of the waist feature to gather and form ruffles or rugosities having ridges and valleys generally transverse to the lengths of the elastic members 228, and extending in the z-direction.

In further embodiments, to adhere the components of the waist feature laminate, the elastic members may be individually coated with adhesive ("strand coated") prior to incorporation into the waist laminate. Various coating methods and techniques, including strand coating methods and techniques, are shown for example in U.S. Pat. Nos. 5,340,648; 5,501,756; 5,507,909; 6,077,375; 6,200,635; 6,235,137; 6,361,634; 6,561,430; 6,520,237; 6,582,518; 6,610,161; 6,613,146, 6,652,693, 6,719,846 and 6,737,102. The adhesive used may be a hot-melt type adhesive having elasticity and flexibility making it suitable for attaching pre-strained elastic materials to substrates, such as OMNIMELT BLOCKS 22 H2401F, or ZEROCREEP brands such as AVANCE, available from Bostik, Inc., Wauwatosa, Wisconsin. The adhesive may comprise a colored adhesive 26 having a colorant 28 as described herein.

In certain embodiments, corners of the front and/or rear belt portion may be trimmed off as suggested in Fig. 7b. The corners may be trimmed off along straight lines, or may be trimmed off along trim paths that are curved and either concave or convex with respect to the remaining area of the belt portion (*see* Fig. 7b), as may be desired to impart a particular curved leg edge profile. In conjunction with such trimming and the configuration of elastic strands described above, it may be desired to impart bonding between layers along edges of the respective belt portion 222, 223. Such bonding may serve to prevent any separation of the layers along edges that may contribute to creating a ragged appearance, and may also help the rear belt portion more effectively draw inward laterally toward the central chassis 122, under the contractive force of the elastic strands below seams 224. Bonding may be effected by mechanical/compression bonds as described in, for example, U.S. Pats. Nos. 4,854,984 and 4,919,738, by thermal bonds or welds, or by deposits of adhesive between layers. In nonlimiting examples, such bonding may form a pattern along edges. Such bonding may be supplemental to any bonding between layers generally holding the respective belt portion 222, 223 together as a laminate structure.

Side seams 224 may be permanent or refastenable. Permanent seams may be formed between the front belt portion and the rear belt portion by any bonding mechanism wherein the front and rear belt portions may not be forcibly separated without substantial damage to one or both of the front and rear belt portions, or without any included mechanism by which substantial reattachment or refastening may be effected. Bonding forming permanent seams may include compression bonding, thermal bonding/welds, ultrasonic bonding or adhesive bonding. Refastenable seams may be formed between the front belt portion and the rear belt portion by any mechanism configured to permit substantially non-destructive forcible separation of the front and rear belt portions, and subsequent substantial reattachment or refastening at the same locations. One example of such mechanism is a hook-and-loop fastening system, for example, a VELCRO fastening system. A suitably sized and shaped hooks component may be bonded to one of the front or rear belt portions along the longitudinal edges thereof, and a suitably sized and shaped loops component may be bonded to the other of the front or rear belt portions along the longitudinal edges thereof, in positions in which they may be brought together and engaged to form seams 224. Examples are depicted in U.S. Pat. App. Serial Nos. 61/787,416; 61/787,332; 61/666,065.

Exemplary belt and absorbent pant constructions are disclosed in U.S. Pat. App. Nos. 14/598,783 and 14/032,595.

In certain embodiments, the belt 220 or a portion of the belt comprises one or more printed region 12. The printed region 12 may comprise one or more colorants 28, which may be disposed in one or more patterns 24 and/or design elements 22 including but not limited to graphics, instructional indicia, and insignia. Additionally, or alternatively, the inspection zone may comprise the belt or a portion of the belt, including the seam 224 or a portion of the seam.

PACKAGE

The absorbent articles 100 of the present disclosure may be placed into packages. The packages may comprise polymeric films and/or other materials. Graphics and/or indicia relating to properties of the absorbent articles may be formed on, printed on, positioned on, and/or placed on outer portions of the packages. Each package may comprise a plurality of absorbent articles. The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

Accordingly, packages of the absorbent articles of the present disclosure may have an In-Bag Stack Height of less than about 110 mm, less than about 105 mm, less than about 100 mm, less than about 95 mm, less than about 90 mm, less than about 85 mm, less than about 80 mm, less than about 78 mm, less than about 76 mm, less than about 74 mm, less than about 72 mm, or less than about 70 mm, specifically reciting all 0.1 mm increments within the specified ranges and all ranges formed therein or thereby, according to the In-Bag Stack Height Test described herein. Alternatively, packages of the absorbent articles of the present disclosure may have an In-Bag Stack Height of from about 70 mm to about 110 mm, from about 70 mm to about 105 mm, from about 70 mm to about 100 mm, from about 70 mm to about 95 mm, from about 70 mm to about 90 mm, from about 70 mm to about 85 mm, from about 72 mm to about 80 mm, or from about 74 mm to about 78 mm, specifically reciting all 0.1 mm increments within the specified ranges and all ranges formed therein or thereby, according to the In-Back Stack Height Test described herein.

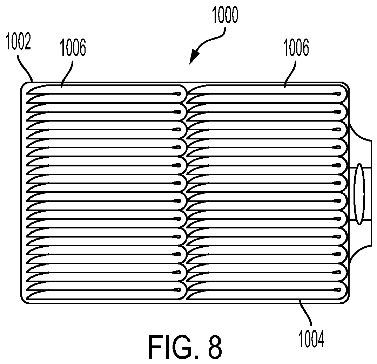

Fig. 8 illustrates an example package 1000 comprising a plurality of absorbent articles 1004. The package 1000 defines an interior space 1002 in which the plurality of absorbent articles 1004 are situated. The plurality of absorbent articles 1004 are arranged in one or more stacks 1006.

Test Methods

Percent Printed Color Area

Percent Printed Color Area is used to determine the amount of printed color coverage on a component layer of an absorbent article images acquired using a flatbed scanner. The scanner is capable of scanning in reflectance mode at a resolution of 150 dpi and 24 bit color (a suitable scanner is an Epson Perfection V750 Pro from Epson America Inc., Long Beach CA or equivalent). The scanner is interfaced with a computer running an image analysis program (a suitable program is ImageJ v. 1.50 or equivalent, National Institute of Health, USA). The specimen images are distance calibrated against an acquired image of a ruler certified by NIST. The resulting image is then analyzed using the image analysis program to identify the boundaries of the printed color regions and calculate the percent printed color area.

Remove the printed substrate of interest from an absorbent article using cryogenic spray (such as Cyto-Freeze, Control Company, Houston TX) or other means as needed to separate the substrate from other components of the article and avoid any longitudinal and lateral distortion of the specimen. Five replicates of this specimen layer, obtained from five substantially similar absorbent articles, are prepared for analysis. Precondition the specimens at about 23 °C $\pm$ 2 C° and about 50% $\pm$ 2% relative humidity for 2 hours prior to testing.

Place the ruler on the center of the scanner bed, oriented parallel to the sides of the scanner glass, and close the lid. Acquire a calibration image of the ruler in reflectance mode at a resolution of 150 dpi (approximately 5.9 pixels per mm) and 24 bit color. Save the calibration image as an uncompressed TIFF format file. Lift the lid and remove the ruler. After obtaining the calibration image, all specimens are scanned under the same conditions and measured based on the same calibration file. Next, place the specimen onto the center of the scanner bed, lying flat, with the color printed facing surface of the specimen facing the scanner's glass surface. Cover the specimen with a white background (in this test method white is defined as having L* > 94, -2 < a* < 2, and -2 < b* < 2 based on the standard CIE L*a*b* color space) and close the lid. Acquire and save a scanned image of the specimen layer. If the size of the specimen layer exceeds the available scanning area, obtain multiple scans covering the entire specimen layer and digitally stitch them together into a single image for analysis. Scan the remaining four replicates in like fashion.

Open the calibration image file in the image analysis program and perform a linear distance calibration using the imaged ruler. This distance calibration scale will be applied to all subsequent specimen images prior to analysis. Open a specimen image in the image analysis program and set the distance scale. Using the image analysis program, identify and define the boundaries of any printed color regions in the image of the specimen layer. Identification of color region boundaries should be performed with the intent of defining them as they would be discerned by a human viewer under standard lighting conditions with the unaided eye if the layer were being viewed face on in a flat configuration at approximately an arm's length distance. For example, intra-dot spaces commonly associated with ink-jet printing are included within that ink region, because they are perceived as part of that printed region by a typical viewer without magnification.

Calculate the area of each of the individual printed color regions within the image to the nearest 0.1 mm$^2$. Calculate the total area of printed color by summing up the areas of the individual printed color regions. Divide the total area of the printed color regions by the area of the entire specimen layer and multiply by 100. Record this value as the printed color percent area to the nearest 0.1%. In like fashion, analyze the remaining four specimen images. Calculate and report the average printed color percent area to the nearest 0.1% for the five replicates.

Color Method

Color analyses are made using a 45° circumferential illumination/0° viewing spectrophotometer capable of making standard CIE L*a*b* measurements in accordance with ASTM E1349 (a suitable instrument is the eXact spectrophotometer, available from X-Rite, Grand Rapids MI, or equivalent). The instrument is configured with a port diameter of 4.0 mm or other diameter appropriate for the size of the region to be measured, such that only the printed site of interest is observed within the port. Set the instrument parameters to 2° Observer, Illumination C, Density Measurement Condition M0 (no filter, UV included), Absolute Density White Balance, and ISO (ANSI) Reflectance Status T. A White Standard Board, available as PG2000 from Sun Chemical-Vivitek Division, Charlotte, NC or equivalent, is used as a specimen backing. Analyses are performed in a room controlled at about 23 °C $\pm$ 2 C° and 50 % $\pm$ 2 % relative humidity. Samples and the White Standard Board are conditioned at the same condition for 2 hours before testing. Calibrate the instrument per the vender's instructions.

Remove the printed substrate of interest from the article. Determine the side on which the printed ink was applied and make all measures on that surface. Select the printed site to be measured. Place the specimen, printed side facing upward onto the Standard White Board. Align the measurement port of the instrument on the specimen, ensuring only the printed site of interest is visible within the port. Take a reading of L*a*b* and record each value to the nearest 0.01 units.

In like fashion, take three color measurements on equivalent sites on three replicate samples. Calculate the arithmetic mean of the color values for the replicates and report each to the nearest 0.01 units.

Topsheet AGM Residue Method

The Topsheet AGM Residue (TAGMR) Method is used to assess the presence of absorbent gelling materials (AGM) particles on the skin-facing side of the topsheet of an absorbent article. In this method, like samples of absorbent articles are stretched flat with skin-facing side facing up and are misted with aqueous copper sulfate solution. AGM particles are colored blue by the copper sulfate, and the prevalence of AGM particles is visually assessed. All testing is conducted in a conditioned room at a temperature of 23° C $\pm$ 2.0° C and a relative humidity of 45% $\pm$ 10% on samples that have been conditioned for a minimum of 12 hours to these same conditions prior to the test.

Ten nominally equivalent samples of absorbent articles are selected at random. For each sample article, the elastic leg cuffs are removed, and the sample is held flat on a board outfitted with adhesive or hook material. With a handheld pump sprayer, 15 $\pm$ 5 mL of a 0.5% w/w aqueous solution of copper sulfate is sprayed approximately uniformly across the topsheet of the article. Within 1 minute of delivery of this solution, the topsheet is visually assessed, and the number of distinct AGM particles observed on the topsheet is recorded.

The arithmetic mean of number of particles observed across the ten samples is calculated and reported to the nearest integer as the Average Number of Particles Per Pad. The number of samples for which no particles were observed is divided by 10 (the total number of samples) and reported as a percentage as the Percentage of Pads with No Residue Particles.

In-Bag Stack Height Test

The in-bag stack height of a package of absorbent articles is determined as follows:

Equipment

A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within $\pm$ 0.5 mm. The horizontal sliding plate and the horizontal base plate are larger than the surface of the absorbent article package that contacts each plate, i.e. each plate extends past the contact surface of the absorbent article package in all directions. The horizontal sliding plate exerts a downward force of 850 $\pm$ 1 gram-force (8.34 N) on the absorbent article package, which may be achieved by placing a suitable weight on the center of the non-package-contacting top surface of the horizontal sliding plate so that the total mass of the sliding plate plus added weight is 850 $\pm$ 1grams.

Test Procedure

Absorbent article packages are equilibrated at 23 $\pm$ 2 °C and 50 $\pm$ 5 % relative humidity prior to measurement.

The horizontal sliding plate is raised and an absorbent article package is placed centrally under the horizontal sliding plate in such a way that the absorbent articles within the package are in a horizontal orientation (see Fig. 8). Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within $\pm$ 0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) $\times$ 10 is calculated and reported to within $\pm$ 0.5 mm.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

**Claims**

1. A method of manufacturing an absorbent article having a printed region **characterized by** the steps:

   printing an article component in an inspection zone with a colorant wherein the colorant is fluorescent-free and comprises carbon black, and wherein the colorant comprises a L* value of 85 or less;
   passing the inspection zone through an infrared inspection system.

2. The method according to claim 1 wherein the colorant comprises 0.01% to about 0.5% of carbon black pigment by weight of the colorant.

3. The method according to claims 1 or 2 wherein the infrared inspection system comprises an infrared light source emitting light in the range of 700 nm -1200 nm wavelength.

4. The method according to claim 3 wherein the infrared inspection system comprises a near-infrared light source emitting light in a range of 700-1000 nm wavelength.

5. The method according to any of the preceding claims wherein the colorant further comprises a second component, wherein the second component is non-IR absorbing.

6. The method according to any of the preceding claims further comprising using the infrared inspection system to detect positioning of the article component.

7. The method according to any of the preceding claims wherein the colorant is within the boundary described by the following system of equations:

$$\{a^* = -1 \, to \, 3; b^* = 12 \, to \, 6\} \rightarrow b^* = -1.5a^* + 10.5$$

$$\{a^* = 3 \, to \, 7; b^* = 6 \, to \, 4\} \rightarrow b^* = -0.5a^* + 7.50$$

$$\{a^* = 7 \, to \, 8; b^* = 4 \, to \, 0\} \rightarrow b^* = -4 \, a^* + 32$$

$$\{a^* = 8 \, to \, 0; b^* = 0 \, to -10\} \rightarrow b^* = 1.25a^* - 10$$

$$\{a^* = 0 \, to -7; b^* = -10 \, to -8\} \rightarrow b^* = -0.29a^* - 10$$

$$\{a^* = -7 \, to -9; b^* = -8 \, to \, 1\} \rightarrow b^* = -4.5a^* - 39.5$$

$$\{a^* = -9 \, to -1; b^* = 1 \, to \, 12\} \rightarrow b^* = 1.375a^* + 13.38$$

wherein L* is 20 to 50.

8. The method according to any of claims 1-6 wherein the colorant is within the boundary described by the following system of equations:

$$\{a^* = -4 \, to \, 4; b^* = 14 \, to \, 7\} \rightarrow b^* = -0.875a^* + 10.5$$

$$\{a^* = 4 \ to \ 5; b^* = 7 \ to - 2\} \rightarrow b^* = -9a^* + 43$$

$$\{a^* = 5 \ to \ 1.5; b^* = -2 \ to - 12\} \rightarrow b^* = 2.9 \ a^* - 16.3$$

$$\{a^* = 1.5 \ to - 4; b^* = -12 \ to - 10\} \rightarrow b^* = -0.36a^* - 11.5$$

$$\{a^* = -4 \ to - 5; b^* = -10 \ to - 4\} \rightarrow b^* = -6a^* - 34$$

$$\{a^* = -5 \ to - 4; b^* = -4 \ to \ 14\} \rightarrow b^* = 18a^* + 86$$

wherein L* is 51 to 70

9. The method according to any of the preceding claims wherein the colorant comprises an a* value of from about -9 to about 9.

10. The method according to claim 9 wherein the a* value is between -1 and 2.

11. The method according to any of the preceding claims wherein the colorant further comprises a b* value of -10 to 10.

12. The method according to claim 11 wherein the b* value is between -4 and 5.

13. The method according to any of the preceding claims wherein the article component is selected from the group consisting of: a backsheet, a fastening system, a landing zone, an ear, a leg cuff, a waistband, a core wrap, a belt, and combinations thereof.

14. The method according to any of the preceding claims wherein further comprising printing a pattern with the colorant.

15. An absorbent article comprising the article component printed by the method according to any of the preceding claims.

FIG. 1

FIG. 2

FIG. 3A

FIG. 3B

L is 20 through 50

FIG. 4A

L is 51 through 70

FIG. 4B

FIG. 5

FIG. 6

FIG. 7A

FIG. 7B

FIG. 8

Europäisches
Patentamt
European
Patent Office
Office européen
des brevets

# EUROPEAN SEARCH REPORT

Application Number

EP 19 21 7286

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/361207 A1 (MISEK JENNIFER L [US] ET AL) 15 December 2016 (2016-12-15) * paragraph [0068]; figures 6(b),7(b) * * paragraph [0068] * * paragraphs [0081], [0089]; table 2 * * paragraph [0067]; figure 5 * * figure 1 * | 1-15 | INV. A61F13/15 A61F13/514 A61F13/84 G01N21/35 |
| A | EP 2 491 900 A1 (UNI CHARM CORP [JP]) 29 August 2012 (2012-08-29) * paragraph [0055] * | 15 | |
| A | WO 00/07426 A2 (PROCTER & GAMBLE [US]) 17 February 2000 (2000-02-17) * table V * | 1-15 | |

TECHNICAL FIELDS
SEARCHED (IPC)

A61F
G01N

The present search report has been drawn up for all claims

| Place of search | Date of completion of the search | Examiner |
|---|---|---|
| Munich | 7 April 2020 | Beckert, Audrey |

EPO FORM 1503 03.82 (P04C01)

## ANNEX TO THE EUROPEAN SEARCH REPORT
## ON EUROPEAN PATENT APPLICATION NO.

EP 19 21 7286

This annex lists the patent family members relating to the patent documents cited in the above-mentioned European search report.
The members are as contained in the European Patent Office EDP file on
The European Patent Office is in no way liable for these particulars which are merely given for the purpose of information.

07-04-2020

| Patent document cited in search report | | | Publication date | Patent family member(s) | | | Publication date |
|---|---|---|---|---|---|---|---|
| US 2016361207 | A1 | | 15-12-2016 | AU | 2010308100 | A1 | 05-04-2012 |
| | | | | CN | 102573742 | A | 11-07-2012 |
| | | | | EP | 2488142 | A2 | 22-08-2012 |
| | | | | KR | 20120099405 | A | 10-09-2012 |
| | | | | RU | 2012119825 | A | 27-11-2013 |
| | | | | US | 2011088828 | A1 | 21-04-2011 |
| | | | | US | 2016361207 | A1 | 15-12-2016 |
| | | | | WO | 2011045683 | A2 | 21-04-2011 |
| EP 2491900 | A1 | | 29-08-2012 | AU | 2010309092 | A1 | 31-05-2012 |
| | | | | CN | 102573712 | A | 11-07-2012 |
| | | | | EP | 2491900 | A1 | 29-08-2012 |
| | | | | JP | 5396234 | B2 | 22-01-2014 |
| | | | | JP | 2011087825 | A | 06-05-2011 |
| | | | | US | 2012245541 | A1 | 27-09-2012 |
| | | | | WO | 2011049010 | A1 | 28-04-2011 |
| WO 0007426 | A2 | | 17-02-2000 | AR | 022060 | A1 | 04-09-2002 |
| | | | | AU | 741603 | B2 | 06-12-2001 |
| | | | | BR | 9906727 | A | 21-11-2000 |
| | | | | CA | 2305169 | A1 | 17-02-2000 |
| | | | | CN | 1286609 | A | 07-03-2001 |
| | | | | CO | 5100952 | A1 | 27-11-2001 |
| | | | | DE | 29923521 | U1 | 07-12-2000 |
| | | | | GB | 2347430 | A | 06-09-2000 |
| | | | | HU | 0004384 | A2 | 28-06-2001 |
| | | | | ID | 28184 | A | 10-05-2001 |
| | | | | JP | 2002521169 | A | 16-07-2002 |
| | | | | KR | 20010024444 | A | 26-03-2001 |
| | | | | MY | 118382 | A | 30-10-2004 |
| | | | | PE | 20001039 | A1 | 23-12-2000 |
| | | | | TR | 200002000 | T1 | 21-10-2005 |
| | | | | TW | 495574 | B | 21-07-2002 |
| | | | | US | 6096412 | A | 01-08-2000 |
| | | | | WO | 0007426 | A2 | 17-02-2000 |
| | | | | ZA | 200001749 | B | 22-02-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6120487 A
- US 20140148773 A
- US 20140148774 A
- US 20140144579 A
- US 8674006 A
- US 9217070 B
- US 8145344 B
- US 8145343 B
- US 8145338 B
- US 9910429 B
- US 3860003 A
- US 5151092 A
- US 5221274 A
- US 5554145 A
- US 5569234 A
- US 5580411 A
- US 6004306 A
- US 5607760 A
- US 5609587 A
- US 5635191 A
- US 5643588 A
- US 4892536 A
- US 4990147 A
- US 5037416 A
- US 5269775 A
- US 4610678 A
- US 5260345 A
- US 5387207 A
- US 5397316 A
- US 5625222 A
- US 8979815 B
- US 9060904 B
- US 9072634 B
- US 491642
- WO 9516746 A
- US 5865823 A
- US 5571096 A
- US 6107537 A
- US 5137537 A
- US 5549791 A
- WO 9534329 A
- US 2007118087 A
- US 20080312622 A1
- US 7786341 B
- WO 200059430 A
- WO 9510996 A
- US 5700254 A
- WO 02067809 A
- US 2015065973 A
- US 3848594 A
- US 4662875 A
- US 4846815 A
- US 4894060 A
- US 4946527 A
- US 6432098 B
- US 4963140 A
- US 4699622 A
- US 5242436 A
- US 5499978 A
- US 5507736 A
- US 5591152 A
- US 6863666 B
- US 6132411 A
- US 7870652 B
- US 8992499 B
- US 8690852 B
- US 8382736 B
- US 62134622
- US 14077708 B
- US 8939957 B
- US 7435243 B
- US 8062279 B
- US 490543
- US 14533472 B
- US 62134622 B
- US 5266392 A
- US 6645569 B
- US 6863933 B
- US 7112621 B
- US 338603
- US 10338610 B
- US 13005237 B
- US 5382400 A
- US 5418045 A
- US 5707468 A
- US 6454989 B
- US 6632386 B
- US 5622772 A
- US 7291239 B
- US 20100040826
- US 5340648 A
- US 5501756 A
- US 5507909 A
- US 6077375 A
- US 6200635 B
- US 6235137 B
- US 6361634 B
- US 6561430 B

- US 6520237 B
- US 6582518 B
- US 6610161 B
- US 6613146 B
- US 6652693 B
- US 6719846 B
- US 6737102 B
- US 4854984 A
- US 4919738 A
- US 61787416
- US 61787332 B
- US 61666065 B
- US 598783
- US 14032595 B